(19) Europäisches Patentamt
European Patent Office
Office européen des brevets

(11) Publication number: **0 033 241**
**A2**

(12) **EUROPEAN PATENT APPLICATION**

(21) Application number: 81300323.3

(22) Date of filing: 23.01.81

(51) Int. Cl.³: A 61 K 9/02
A 61 K 31/43, A 61 K 31/545

(30) Priority: 25.01.80 JP 8032/80

(43) Date of publication of application:
05.08.81 Bulletin 81/31

(84) Designated Contracting States:
BE CH DE FR GB IT LI NL SE

(71) Applicant: Takeda Yakuhin Kogyo Kabushiki Kaisha
27, Doshomachi 2-Chome
Higashi-ku Osaka, (541)(JP)

(72) Inventor: Ogawa, Yasuaki
32-503, 7 Nakahozumi 1-chome
Ibaraki, Osaka 567(JP)

(72) Inventor: Morita, Yasuhiko
314-108, 6 Otokoyama-sasadani
Yahata, Kyoto 614(JP)

(72) Inventor: Yamaoka, Takashi
14-3, Nishifukui 3-chome
Ibaraki, Osaka 567(JP)

(74) Representative: Myerscough, Philip Boyd et al,
J.A.Kemp & Co. 14, South Square Gray's Inn
London, WC1R 5EU(GB)

(54) **Suppository base and pharmaceutical compositions for rectal administration, and process for their production.**

(57) A suppository base composition for rectal administration comprises a fatty or oily base and polyoxyethylene cholesterol ether and/or polyoxyethylene hydrogenated cholesterol ether. The suppository base is suitable for pharmaceutical preparations for rectal administration containing a compound having a $\beta$-lactam ring, e.g. penicillins and cephalosporins.

EP 0 033 241 A2

Croydon Printing Company Ltd.

0033241

"SUPPOSITORY BASE AND PHARMACEUTICAL COMPOSITIONS FOR RECTAL ADMINISTRATION, AND PROCESS FOR THEIR PRODUCTION"

The present invention relates to a suppository base composition for rectal administration for a useful compound, for example, compounds having a β-lactam ring.

Compounds having a β-lactam ring, inclusive of antibiotics such as penicillins and cephalosporins, exhibit a broad spectrum of antimicrobial activity, and are known to be therapeutic agents for a variety of microbial infections. Administration by injection or/and oral administration is the usual method of administration adopted conventionally for these antibiotics. Administration by injection is superior in terms of *in vivo* absorption of active ingredients but accompanied with drawbacks such as shortened duration of high concentration level in bloods and pain inherently involved. On the other hand, oral administration leads in many cases to poor bioavailability and is almost by means of capsules in the dosage form, thus suffering from disadvantages such as being difficult for children to swallow.

In order to overcome these disadvantages, compositions adapted for administration into the rectum have been proposed, but such antibiotics as penicillins and cephalosporins can hardly be absorbed into body merely by their rectal administration in such dosage forms as dispersed in a fatty, oily or aqueous base. Under such circumstances, a method has been developed, for example, comprising incorporating a nonionic surfactant such as polyoxyethylene lauryl alcohol ether and the like together with a base to increase absorption of the particular penicillin or cephalosporin (U.S. patent 3881012 and French patent 2275214). However, even by the use of a composition prepared by the method as above, the concentration level of antibiotics in blood is still lower than that attained by injection. When a higher concentration level is desired, the amount of

addition of the surfactant must be increased, which leads to the intensified local irritation to inevitably cause defecation or expulsion of suppositories.

Explanation is made with respect to a β-lactam-ring compound hereinafter. However, it should be understood that the present suppository base can be similarly applied to any other useful compounds in place of the β-lactam ring compound.

The present inventors found that by adding polyoxyethylene cholesterol ether or polyoxyethylene hydrogenated cholesterol ether to a composition containing a β-lactam-ring compound being generally less absorbable upon rectal administration and a fatty or oily base, there is obtained the composition permitting very fast absorption of the β-lactam-ring compound through the rectum, not showing side effects such as promotion of defecation and maintaining high concentration levels of the drug substance in blood for a longer period of time than administration in the injection dosage form, and have come to complete the present invention.

Thus, the present invention relates to compositions for rectal administration which contain compounds having a β-lactam-ring, polyoxyethylene cholesterol ether or/and polyoxyethylene hydrogenated cholesterol ether and an oily base.

The compounds having a β-lactam ring according to the present invention include, for example, the penicillin and cephalosporin compounds of the general formula:

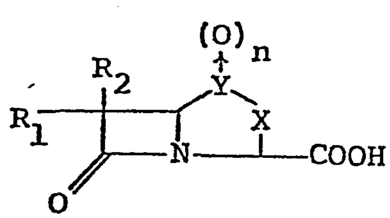

[wherein $R_1$ is an acylamide or amidino group; $R_2$ is a hydrogen atom or a lower alkoxy group; X represents 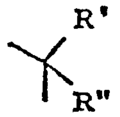 or 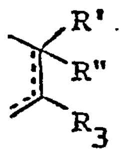 (where R' and R" are the same or different, and each means a hydrogen atom or a methyl group; $R_3$ is a hydrogen atom, lower alkyl (e.g. methyl, ethyl, etc.), halogen (e.g. chlorine, etc.), formyl or a group: $-CH_2R$ or $-CH=CH-R$ (R is hydroxyl; acyloxy such as acetoxy; carbamoyloxy; lower alkoxyl such as methoxy; quaternary ammonium group which may be substituted; or heterocyclicthio group which may be substituted); Y represents an oxygen or sulfur atom; n is an integer of 0 to 2]. Examples of the acylamide group represented by $R_1$ include organic carboxylic acid acylamide groups, while the acyl group is particularly exemplified by the groups represented by:

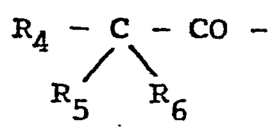

(wherein $R_4$ is alkyl, alkylthio, phenyl, thienyl, thiazol-4-yl, cyclohexadienyl, pyridylthio, tetrazolyl, furyl or cyano which may be substituted by, for example, $NH_2$, OH, CN, $CONH_2$ and COOH, e.g., 4-hydroxyphenyl, 2-aminothiazolyl, cyanomethyl and cyanomethylthio; $R_5$ and $R_6$ both are a hydrogen atom, or one of them is a hydrogen atom and the other amino, sulfo, carboxyl, aminocarbonylamino or hydroxyl, or $R_5$ and $R_6$ combine to represent lower alkoxyimino (e.g., methoxyimino, etc.), hydroxyimino or 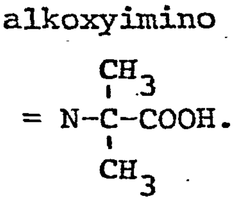.

As the heterocyclic thiomethyl group in the group represented by $R_3$, frequent use is made of the groups where the heterocyclic ring is tetrazolyl, thiadiazolyl, triazolyl, diazolyl, thiazolyl, oxadiazolyl, oxazolyl, 4,5-dihydro-1,2,4-triazinyl or tetrazolo[1,5-b]pyridazinyl and said heterocyclic ring has substituent groups such as lower alkyls, e.g. methyl and ethyl, amino, hydroxyl, thiol, oxo, carboxy lower alkyls, sulfo-lower alkyls, amino lower alkyls and substituted amino lower alkyls, for example, mono- or di-lower alkylamino-lower alkyl (e.g. dimethylaminoethyl, etc.). Acyloxy of the acyloxymethyl represented by the symbol $R_3$ includes, for example, an alkylcarbonyloxy group of 2 to 4 carbon atoms such as acetyloxy or propionyloxy, an acetyloxy group substituted by an alkylcarbonyl group of 2 to 4 carbon atoms such as acetoacetyloxy or propionylacetyloxy, a phenylacetyloxy group which may be substituted in the α-position, for example by hydroxy, sulfo or amino, such as mandeloxy, α-sulfophenylacetyloxy, glycyloxy or phenylacetyloxy, an alkylcarbonyloxy group of 2 to 4 carbon atoms as substituted by a carboxyl group such as succinoyloxy, a group of the general formula:

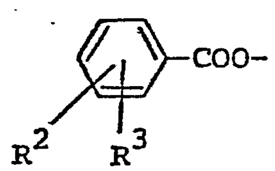

[wherein $R^2$ and $R^3$, respectively, mean hydrogen, carboxyl, carboethoxycarbamoyl, carboethoxysulfamoyl or nitro] such as 2-carboxybenzoyloxy, 2-(carboethoxycarbamoyl)benzoyloxy, 2-(2-carboethoxysulfamoyl)benzoyloxy, 2-carboxy-3(or 4 or 6)-nitrobenzoyloxy or 2,4-dicarboxybenzoyloxy. Quaternary ammonium group in the quaternary ammonium methyl group

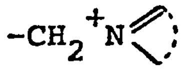 in the symbol $R_3$ may be, for instance, a quaternary ammonium group of the general formula:

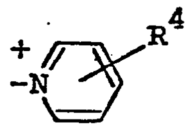

(wherein $R^4$ is hydrogen, methyl, carbamoyl, carboxyl, sulfonic or methoxy) which may be derived from pyridine derivatives such as pyridine, carbamoyl-substituted pyridine such as nicotinamide and isonicotinamide, carboxyl-substituted pyridine such as nicotinic acid and, isonicotinic acid, and sulfonic acid-substituted pyridine such as pyridine-sulfonic acid.

Furthermore, the cephem compounds may have a 3-substituent selected from the group consisting of hydrogen, lower alkyl, chlor or a group of the formula: $-CH_2R$ or $-CH=CHR$ wherein R is acetoxy; carbamoyloxy; lower alkoxyl; pyridynium; 3- or 4-carbamoylpyridynium; 2-methylpyrazolium; or diazolylthio, triazolylthio, tetrazolylthio, thiadiazolylthio, 4, 5-dihydro-1,2,4-triazinylthio or tetrazolo-[1,5-b]pyridazinyl which may be substituted with lower alkyl, carboxymethyl, hydroxyl, amino, mono- or di-lower alkylamino-lower alkyl, oxo or sulfo-lower alkyl.

Examples of such compounds include penicillin antibiotics such as ampicillin, amoxicillin, carbenicillin, sulbenicillin, cyclacillin, mecillinam, ticalcillin, propicillin and 6-[2-(4-ethyl-2,3-dioxo-l-piperadinocarboxyamido)-2-phenylacetamido]-2,2-dimethyl penam-carboxylic-acid, and cefalexin, cefaradine, cephaloglycin, cefatrizine, cefaclor, cephalothin, cephaloridine, cefazolin, cefuroxime, cefacetrile, cefsulodin, cefotiam, cefoxitin, cefmetazole, cefmenoxime, cefotaxime (HR-756), cephthizoxime (FK-749), cephoperazone, cephapirin, ceftezole, cefamandole, cefotietan, (6R, 7R)-7-[2-carboxy-2-(4-hydroxyphenyl)acetamido]-7-methoxy-3-[(l-methyl-lH-tetrazol-5-yl-thio)methyl]-8-oxo-5-oxa-l-azabicyclo[4.2.0]-oct-2-ene-2-carboxylic acid, cefadroxil, pivaloyloxymethyl 7-[2-(2-aminothiazol-4-yl)-2-(<u>syn</u>)-methoxyiminoacetamido]-desacetoxycephalosporanate, (6R, 7R)-7-[(Z)-2-(2-aminothiazol-4-yl)-2-(2-carboxyprop-2-oxyimino) acetamido]-3-(l-pyridyniummethyl)-ceph-3-em-4-carboxylate, 7-[2-(2-aminothiazol-4-yl)-2-(Z)-methoxyimino acetamido]-3-(4-methyl-5-oxo-6-hydroxy-4,5-dihydro-1,2,4-triazin-3-ylthio) methyl-3-cephem-4-carboxylic acid, (6R,7R)-7-[2-(2-amino-4-thiazolyl)-2-(Z-methoxyimino)acetamido]-3-[[(2,5-dihydro-6-hydroxy-2-methyl-5-oxo-as-triazin-3-yl)thio]methyl]-8-oxo-5-thia-l-azabicyclo[4,2,0]oct-2-ene-2-carboxylic acid, etc. These compounds may be salts with alkali metals such as sodium and potassium, salts with alkaline earth metals such as calcium and magnesium, salts with nitrogen-containing organic compounds such as ammonium, benzylamine, amino acids, l-ephenamine and amino acids, salts with inorganic acids such as hydrochloric acid, phosphoric acid and sulfonic acid, salts with organic acids such as citric acid and succinic acid, and the like. In addition, they may form esters. Such ester residues are, for example, lower alkyl groups, e.g. methyl, ethyl, etc.; lower alkoxymethyl groups, e.g. methoxymethyl, ethoxymethyl,

isopropoxymethyl, α-methoxyethyl, α-ethoxyethyl, etc.; α-lower alkoxy-α-substituted methyl groups such as α-lower alkoxy($C_{1-4}$) ethyl (e.g. methoxyethyl, ethoxyethyl, propoxyethyl, iso-propoxyethyl), etc.; lower alkylthiomethyl groups of 1 to 3 carbon atoms, e.g. methylthiomethyl, ethylthiomethyl, isopropylthiomethyl, etc.; acyloxymethyl groups, e.g. pivaloyloxy methyl, α-acetoxymethyl, etc.; ethoxycarbonyloxy-1-methylmethyl; or α-acyloxy-α-substituted methyl groups (e.g. α-acetoxy-α-methylmethyl). Examples of the compounds having a β-lactam ring according to the present invention, which are suitably employed, include the above-mentioned antibiotics such as penicillins and cephalosporins, but are not limited to those of the general formula described above; they may be other penicillin and cephalosporin compounds and, furthermore, use may be made of azethidinone derivatives such as nocardin and, further, compounds having a β-lactam ring such as courabranic acid derivatives.

Polyoxyethylene cholesterol ether and polyethylene hydrogenated cholesterol ether are compounds of the formulas mentioned below, respectively.

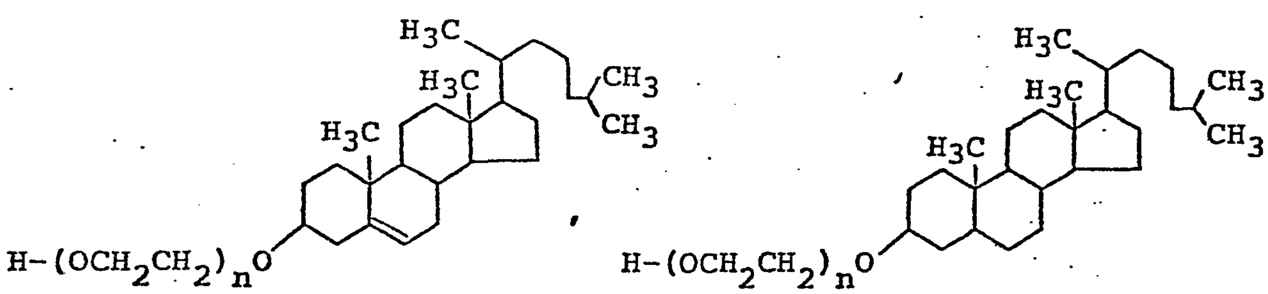

,

Polyoxyethylene cholesterol ether or polyoxyethylene hydrogenated cholesterol ether above is the ether compound prepared by the addition of oxyethylene, as a polyoxyethylene (POE), to cholesterol or 5- and 6-position hydrogenated cholesterol, respectively, and may be used solely or as a mixture thereof. The average number of added moles of POE is normally selected from nearly 5 to 50 moles and, particularly, the adducts having

nearly 10 to 30 moles of POE are especially good for promotion of absorption.

As the fatty or oily excipients of the present invention, use is made of fatty or oily bases which have been concentionally employed for suppositories, etc. As examples of such bases there may be mentioned glycerides of higher fatty acids (e.g., naturally occurring cacao butter, Witepsol produced by Dynamit Nobel AG, a semi-synthetic base), glycerides of medium-chain fatty acids (e.g. Migryol produced by Dynamit Nobel AG), and oils of plant origin (e.g. sesame oil, soybean oil, corn oil, cottonseed oil, etc.). These bases may be employed solely or as a mixture of two or more thereof.

The composition of the present invention is produced, according to the per se conventional manner, by mixing a base additives and a compound having a β-lactam ring and shaping the resulting mass into fatty solid suppositories, semi-solid ointment-like suppositories, capsule suppositories consisting of the liquid composition filled in soft capsules, etc.

A ratio of the compound having a β-lactam ring against the whole composition is generally selected from a range of approximately 0.5 to 50, preferably 5 to 30 weight %, but not limited thereto. The ratio varies depending on the compounds employed, weight of the composition, etc.

The amount of POE cholesterol ether or POE hydrogenated cholesterol to be used is in the proportion of 0.5 to 30 weight %, and preferably 3 to 15 weight %, of the composition.

For purposes of enhancing absorption or controlling the rate of absorption, etc., in the present invention,

any other nonionic surfactant such as polyoxyethylene fatty acid ester and a polyoxyethylene higher alcohol ether may be further incorporated in the composition, or alternatively an anionic surfactant can be used. In addition, a variety of salts or stabilizers can also be incorporated and added in order to increase the solubility or stability of the compound having a β-lactam ring. A dispersing agent, antiseptic agent, etc., besides, can be added, if it is considered necessary from the standpoint of preparation processing.

As the representative examples of the composition of the present invention, there may be mentioned (I) an antibiotic composition for rectal administration containing 5 to 50 weight % of cefsulodin [3-(4-carbamoyl-1-pyridiniomethyl)-7β-(D-α-sulfophenylacetamide)-ceph-3-em-4-carboxylate monosodium salt] and 0.5 to 20 weight % of polyoxyethylene cholesterol ether, (II) an antibiotic composition for rectal administration containing 5 to 50 weight % of cefmenoxime [7β-[2-(2-aminothiazol-4-yl)-[Z]-2-methoxyiminoacetamide]-3-[(1-methyl-1H-tetrazol-5-yl)thiomethyl]-ceph-3-em-4-carboxylate.$1/_2$hydrochloride] and 0.5 to 20 weight % of polyoxyethylene cholesterol ether, (III) an antibiotic composition for rectal administration containing 5 to 50 weight % of mecillinam [6β-[(hexahydro-1H-azepin-1-yl)-methyleneamino]penicillanic acid·sodium salt] and 0.5 to 20 weight % of polyoxyethylene cholesterol ether, and the like.

The composition of the present invention is given into the rectums of warm blooded animals such as a man (particularly, infants), dogs and rats, and, depending upon the compound having a β-lactam ring employed, the dose of the compound having a β-lactam ring etc. can not be unconditionally determined. Generally, it is in the range of 1 to 100 mg/kg/day. In the case of the composition containing cefsulodin (sodium), cefmenoxime (1/2HCℓ) or

mecillinam (sodium), the dose is approximately 1 to 60 mg/kg/day, respectively. The composition for human rectal administration is normally recommended to be given by, for example, the suppository weighing 0.5 to 3.5 g each, twice or three times daily. The rectal administration according to the present invention is highly safe to man and animals.

The Experiment Examples are given in the following.

Percentage means % by weight in Examples below.

Experiment Example 1

Employing the representative compounds having a β-lactam ring, the areas under blood level versus time curves (AUC, Area Under the Curve) were measured with the various control references and the compositions of the present invention, as shown in Table 1.

Suppositories containing different compounds having a β-lactam ring were given, in the form of fatty solid suppositories (150 mg each), into the rectum of SD strain male rats (body weight of 250 to 300 g) being fasted for 15 to 20 hours and anesthetized with a barbital. The anuses were closed with an adhesive agent, and blood samples were taken from the tail veins with time elapsed to determine quantitatively the plasma levels of the compounds having a β-lactam ring by means of the biological assay method. The test microorganism for the assay was varied with type of the compounds having a β-lactam ring, and Bacillus subtilis ATCC 6638, Escherichia coli NIHJ, Pseudomonas aerugionosa NCTC 10490 and Proteus mirabilis ATCC 21100 were employed.

As is apparent from Table 1, rectal administration of the specimens of the present invention afforded by far greater AUC as compared with the cases of rectal

administration of the control specimens, and it was recognized that addition of POE (25) cholesterol produced greatly improved absorption of the compounds having a β-lactam ring. The numeral 25 in the term "POE (25)" designates the average number of added moles of POE.

Table 1: AUC for suppositories of the representative compounds having a β-lactam ring

| | Dose (mg/kg) | Concn.of POE (25) cholesterol / Base | (W/W)% 0 | (W/W)% 3.0 | (W/W)% 5.0 | Intramuscular injection |
|---|---|---|---|---|---|---|
| Ampicillin | 20 | Witepsol W-35 | 0.9 | - | 13.2 | 14.8 |
| Sulbenicillin | 200 | Witepsol W-35 | | 92.8 | - | 192 |
| Mecillinam | 100 | Witepsol W-35 | 4.9 | 79.9 | - | 105.3 |
| Cefalexin | 200 | Witepsol W-35 | 5.7 | - | 58.8 | - |
| Cefacetorile | 100 | Witepsol W-35 | 6.1 | 74.7 | - | - |
| Cefotiam | 40 | (Sodium citrate *1)<br>(Witepsol W-35 | 4.0 | - | 11.8 | 38.7 |
| Cefsulodin sodium | 20 | Witepsol H-5 | 0.5 | - | 35.9 | 54.0 |
| Cefmenoxime (1/2HCℓ) | 50 | (Witepsol W-35<br>(Sodium phosphate *2) | 15.0 | 148.0 | - | 189.7 |
| FK-749 | 50 | Witepsol W-35 | 2.5 | 48.1 | - | 77.8 |
| HR-756 | 50 | Witepsol W-35 | 0.9 | 97.7 | - | 143.9 |
| Cefazolin | 100 | Witepsol W-35 | 0.5 | 203.6 | - | 348.4 |

Notes: *1 Sodium citrate 8%, Witepsol W-35 77%) when no POE(25) cholesterol is added, i.e., 0 W/W%;

Sodium citrate 8%, Witepsol W-35 72%) when 5% of POE(25) cholesterol is added.

*2 Sodium phosphate 1.2%, Witepsol W-35 90.5%) when no POE(25) cholesterol is added;

Sodium phosphate 1.2%, Witepsol W-35 87.5%) when 3 % POE(25) cholesterol is added.

Experiment Example 2

With use of cefmenoxime (Na-salt), the suppository compositions (150 mg each) as shown in Table 2 were prepared, and administered into the rectum of rats, in the same manner as in Experiment Example 1, to determine quantitatively the concentration levels in plasma (plasma levels of cefmenoxime).

Table 2: Concentration levels of cefmenoxime in plasma
dose: 50 mg/Kg

| Route of Administration | | Time (hr) | 0.5 | 1 | 2 | 4 | 6 |
|---|---|---|---|---|---|---|---|
| [Control] Intra-muscular injection | | Aqueous solution of Cefmenoxime | 112.4 | 102.3 | 28.8 | 5.8 | 1> |
| Rectal administration | No.1 | Cefmenoxime; 8.3% Witepsol W-35; 91.7% | 1.9 | 2.1 | 1.4 | 3.2 | 4.1 |
| | No.2 | Cefmenoxime; 8.3% BL-9 (POE(9) Lauryl Alcohol ether); 5.0% Witepsol W-35; 86.7% | 8.4 | 21.0 | 22.3 | 2.8 | 1.6 |
| | No.3 | Cefmenoxime; 8.3% Emalex 523 (POE(23) Oleyl Alcohol ether); 5.0% Witepsol W-35; 86.7% | 26.0 | 35.4 | 26.0 | 3.8 | 2.7 |
| | No.4 | Cefmenoxime; 8.3% BWA-20 (POE(20)Lanolin Alcohol ether); 5.0% Witepsol W-35; 86.7% | 19.8 | 41.4 | 36.1 | 7.9 | 2.2 |
| | No.5 | Cefmenoxime; 8.3% Pluronic L-64 (POE & POP; block polymer); 3.0% Witepsol W-35; 88.7% | 16.0 | 17.6 | 12.8 | 5.2 | 2.0 |
| [Present invention] Rectal administration | | Cofmenoxime; 8.3% POE(24) Cholesterol; 5.0% Witepsol W-35; 86.7% | 79.2 | 84.7 | 41.1 | 8.2 | 4.1 |

Concentration levels in plasma (γ/ml)

With use of cefsulodin (sodium), the suppository compounds (50 mg each) as shown in Table 3 were prepared, administered into the rectum of rats, in the same manner as in Experiment Example 1, to determine quantitatively the concentration levels in plasma.

Table 3: Concentration levels of cefsulodin (sodium) in plasma. dose: 20 mg/Kg

| | Route of adminis-tration | Time (hr) | Concentration levels in plasma (γ/ml) | | | | |
|---|---|---|---|---|---|---|---|
| | | | 0.5 | 1 | 2 | 4 | 6 |
| Control | Intra-muscular injection | Aqueous solution cefsulodin | 29.3 | 21.4 | 16.5 | 5.6 | 3.2 |
| | Rectal adminis-tration | Cefsulodin 10% BL-9 2% Witepsol H-5 88% | 3.9 | 7.8 | 2.4 | 1.1 | 0.4 |
| | " | Cefsulodin 10% BL-25 (POE(25) lauryl alcohol ether) 5% Witepsol H-5 85% | 12.5 | 7.3 | 5.2 | 2.0 | 2.3 |
| | " | Cefsulodin 10% Emalex 630 (POE(30) stearyl alcohol ether) 5% Witepsol H-5 85% | 6.4 | 9.2 | 3.3 | 2.8 | 1.6 |
| | " | Cefsulodin 10% BO-20 (POE(20) oleyl alcohol ether) 5% Witepsol H-5 85% | 14.8 | 10.3 | 2.8 | 2.1 | 1.4 |
| Present inven-tion | Rectal adminis-tration | Cefsulodin 10% POE(25) cholesterol 5% Witepsol H-5 85% | 16.4 | 18.5 | 7.9 | 4.7 | 2.3 |

As is obvious from Tables 2 and 3, the specimen according to the present invention was recognized to show prolonged duration time of the concentration level of cefmenoxime in plasma as compared with the intramuscular injection, and increase in absorption amounts of cefmenoxime and cefsulodin as compared with the cases of control.

## Experiment Example 3

Utilizing cefmenoxime (1/2HCℓ), the suppository compositions as shown in Table 4 were prepared, and administered into the rectum of rats, in the same manner as in Experiment Example 1, to determine quantitatively the concentration levels in plasma, thereby calculating AUC over the period of 0 to 6 hours.

Table 4: $AUC^{\circ}_{6\ hr}$ for cefmenoxime, dose 50 mg/Kg

| POE cholesterol ether or POE hydrogenated cholesterol ether | (W/W)% | $AUC^{\circ}_{6}$ |
|---|---|---|
| POE (25) cholesterol ether | 3.0 | 148.0 |
| POE (25) cholesterol ether | 1.0 | 116.9 |
| POE (30) cholesterol ether | 1.0 | 98.5 |
| POE (20) cholesterol ether | 3.0 | 150.9 |
| POE (25) hydrogenated cholesterol ether | 3.0 | 108.6 |
| None | 0 | 15.0 |
| Intramuscular injection | – | 189.7 |

Remarks; 1.2 % of sodium phosphate and 87.5 to 89.5 % of Witepsol W-35 were contained as miscellaneous ingredients.

As is apparent from Table 4, it was recognized that addition of POE cholesterol ether and POE hydrogenated cholesterol ether, regardless of the number of added moles of POE, led to greatly improved absorption of the compound having a β-lactam ring.

## Experiment Example 4

By incorporating 10 % of cefsulodin (sodium) and 5 % of polyoxyethylene cholesterol ether as shown in Table 5 into Witepsol H-5 used as an excipient, suppositories (50 mg each) were prepared, and administered rectally to rats, in the same manner as in Experiment Example 1, to determine quantitatively the concentration levels of the drug substance in plasma, thereby calculating AUC over the period of 0 to 4

hours. The dosage was 20 mg/Kg.

Table 5:

| Polyoxyethylene cholesterol ether | AUC (0 to 4 hours) |
|---|---|
| POE (10) cholesterol ether | 30.4 |
| POE (20) cholesterol ether | 43.0 |
| POE (25) cholesterol ether | 35.9 |
| POE (30) cholesterol ether | 37.9 |
| None | 0.5 |

The Examples of the present invention are given below.

Example 1

A 2 g portion of POE (25) cholesterol ether and 12.1 g of a higher fatty acid glyceride (Dynamit Nobel AG, Witepsol W-35) were uniformly molten, and 0.75 g of cefmenoxime (1/2HCℓ) and 0.1 g of sodium phosphate were added to the molten mass to disperse uniformly, followed by filling each 2.0 g portion into suppository containers made of plastics and gradually cooling to obtain suppositories.

Example 2

A 1.5 g portion of POE (25) hydrogenated cholesterol ether and 12.6 g of a higher fatty acid glyceride (produced by Dynamit Nobel AG, Witepsol W-35) were uniformly molten, and 0.75 g of cefmenoxime (Na) was evenly dispersed into the molten mass, followed by filling each 1.0 g portion into suppository containers made of plastics and gradually cooling to obtain suppositories.

Example 3

A 4 g portion of polyoxyethylene (24) cholesterol ether was dissolved with warming into 76 g of sesame oil and 20 g of finely divided cefsulodin sodium was uniformly dispersed into the mass, followed by filling each 1 g portion into a 2.5-mℓ injection syringe made of plastics

to produce the preparation form designed for introduction into the rectum.

Example 4

A 5 g quantity of POE (20) cholesterol ether was dissolved with warming into 75 g of a higher fatty acid glyceride (produced by Dynamit Nobel AG, Witepsol H-15), and 20 g of finely divided cefsulodin sodium was uniformly dispersed into the mass, followed by filling each 2.5 g portion into suppository containers made of plastics and gradually cooling to obtain suppositories.

Example 5

A 5 g portion of POE (30) cholesterol ether was admixed for melting with 220 g of Witepsol W-35 (produced by Dynamit Nobel AG), and 25 g of finely divided sulbenicillin (sodium) was uniformly dispersed into the mass, followed by filling each 2.5 g portion into suppository containers made of plastics and gradually cooling to obtain suppositories.

Example 6

A 4.5 g portion of POE (15) hydrogenated cholesterol ether and 8.7 g of a higher fatty acid glyceride (produced by Dynamit Nobel AG, Witepsol W-35) were uniformly molten, and 1.8 g of mecillinam was uniformly dispersed into the molten mass, followed by filling each 2.0 g portion into suppository containers made of plastics and gradually cooling to obtain suppositories.

As described in detail with respect to cephalosporins and penicillins, the present suppository base composition can be applied to any other useful compounds. Ratio of the fatty or oily base to at least one member selected from the group consisting of polyoxyethylene cholesterol ether and polyoxyethylene hydrogenated cholesterol ether may be within the range of 2-100: 1 (parts by weight).

C L A I M S

1. A suppository base composition for rectal administration comprising a fatty or oily base characterised by also comprising polyoxyethylene cholesterol ether and/or polyoxyethylene hydrogenated cholesterol ether.

2. A suppository base composition according to claim 1 wherein the weight ratio of the base to the ether is from 2:1 to 100:1.

3. A pharmaceutical composition for rectal administration which comprises a compound having a β-lactam ring and a fatty or oily base, characterised by also comprising polyoxyethylene cholesterol ether and/or polyoxyethylene hydrogenated cholesterol ether.

4. A pharmaceutical composition as claimed in claim 3 wherein the compound having a β-lactam ring is a penicillin or cephalosporin compound of the formula

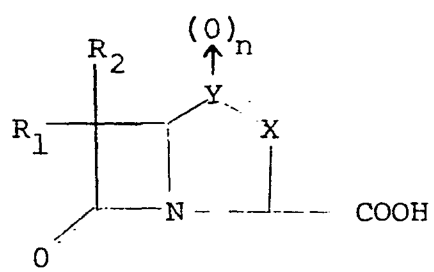

[wherein $R_1$ is an acylamide or amidino group; $R_2$ is hydrogen or lower alkoxy; X represents

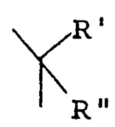 or 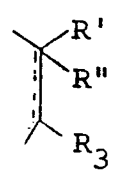 (where R' and R" are the same or different, and each represents hydrogen or methyl; $R_3$ is hydrogen, methyl, formyl, hydroxymethyl, acyloxymethyl, heterocyclic thiomethyl, 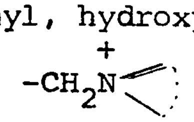

halogen or carbamoyloxymethyl); Y represents an oxygen

or sulfur atom; n is 0, 1 or 2].

5. A pharmaceutical composition as claimed in claim 4, wherein the compound having a β-lactam ring is a cephem compound having 7-acyl group of the formula;

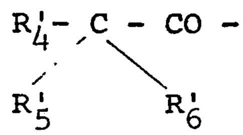

wherein $R_4'$ is thienyl, tetrazolyl, phenyl, thiazolyl, furyl, 4-hydroxyphenyl, cyclohexadienyl, pyridylthio, 2-aminothiazolyl, 2-amino-2-carboxyethylthio, cyanomethyl or cyanomethylthio, $R_5'$ is hydrogen and $R_6'$ is hydrogen, hydroxyl, amino, carboxyl, aminocarbonylamino or sulfo, or $R_5'$ and $R_6'$ jointly represent lower alkoxyimino, hydroxyimino or a group of the formula

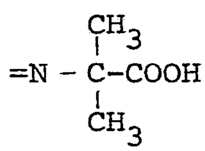 .

6. A pharmaceutical composition as claimed in claim 4, wherein the compound having a β-lactam ring is a cephem compound having 3-substituent selected from hydrogen, lower alkyl, chlorine and a group of the formula: $-CH_2R$ or -CH=CHR wherein R is acetoxy, carbamoyloxy; lower alkoxyl; pyridynium; 3- or 4-carbamoylpyridynium; 2-methylpyrazolium; or diazolylthio, triazolylthio, tetrazolylthio, thiadiazolylthio, 4,5-dihydro-1,2,4-triazinylthio or tetrazolo[1,5-b]pyridazinylthio which may be substituted with lower alkyl, carboxymethyl, hydroxyl, amino, mono- or di- lower alkylamino-lower alkyl, oxo or sulfo-lower alkyl.

7. A pharmaceutical composition as claimed in claim 4 wherein the cephalosporin is cefsulodin, cefotiam or cefmenoxime.

8. A pharmaceutical composition as claimed in any one of claims 3 to 7 wherein an amount of the ether is 0.5 to 30% by weight of the composition.

9. A suppository base composition or pharmaceutical composition as claimed in any one of claims 1 to 8 wherein the polyoxyethylene cholesterol ether and polyoxyethylene hydrogenated cholesterol ether are compounds of the formulae:

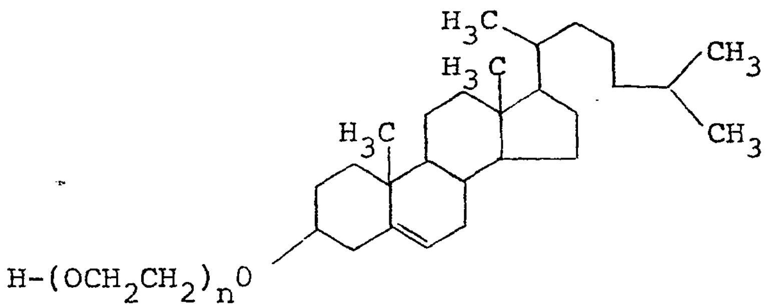

and

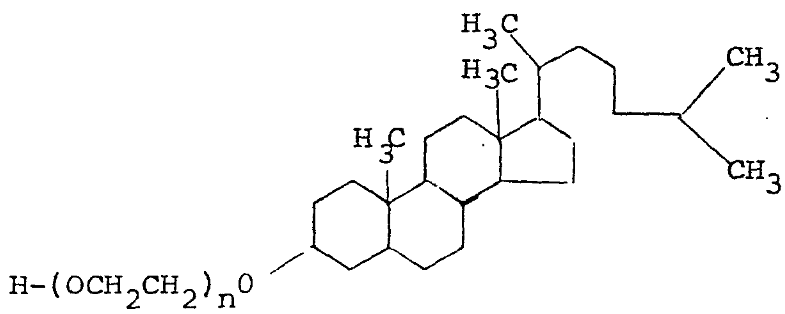

wherein n is 5 to 50, respectively.

10. A method for producing a pharmaceutical composition for rectal administration which comprises mixing a compound having a β-lactam ring and a fatty or oily base, characterised in that a polyoxyethylene cholesterol ether and/or polyoxyethylene hydrogenated cholesterol ether is/are also mixed therewith.